# EUROPEAN PATENT SPECIFICATION

(11) **EP 2 490 749 B1**
(45) Date of publication and mention of the grant of the patent: **14.08.2013**
(21) Application number: 10798584.8
(22) Date of filing: 21.10.2010
(51) Int. Cl.: A61M 37/00

(54) **GUIDE TUBE FOR NEEDLES OF A TATTOO MACHINE**
FÜHRUNGSROHR FÜR TÄTOWIERUNGSMASCHINENNADELN
TUBE DE GUIDAGE CONCU POUR DES AIGUILLES DE MACHINES A TATOUER

(30) Priority: 23.10.2009 IT TO20090808
(43) Date of publication of application: 29.08.2012
(73) Proprietor: Surbone, Andrea, Alpignano (IT)
(72) Inventor: Surbone, Andrea, Alpignano (IT)
(74) Representative: Lovino, Paolo
(86) International application number: PCT/IB2010/002707
(87) International publication number: WO 2011/048482

(56) References cited:
- WO-A2-02/096499
- US-A- 2 743 444
- US-A- 3 228 269
- US-A- 3 979 978
- US-A1- 2003 171 767
- US-A1- 2004 186 501

## Description

### TECHNICAL FIELD

The present invention relates to a guide tube for tattoo machine needles. Such a device is known from WO 02/096499 A2.

### BACKGROUND ART

As known, tattoo machines have a plurality of needles fixed to the end of a supporting rod, which is actuated by an actuating device so as to longitudinally translate with reciprocating motion.

The supporting rod axially extends through a tube comprising a tang fixed to the machine, a tip, from which the needles protrude, and an intermediate gripping portion, which has a diameter normally larger than that of the tip and of the tang. The tang, the tip and the gripping portion may form a single part or be formed by two or more different parts.

The coupling between the tube and the supporting rod, in general, has a given radial clearance, which causes translation inaccuracies, and thus undesired oscillations of the needles in transversal direction. In order to maintain the motion of the needles rectilinear, it is known to exert a force in direction transversal to the supporting rod end which protrudes from the tank, i.e. on the opposite end of the needles. For example, patent US7207242 teaches to use an elastic band to obtain such a transversal forcing.

Such a solution is not completely satisfactory, because the needles are distanced from the point in which the elastic band exerts its withholding force, and consequently such a force is not sufficient to stop the transversal oscillation of the needles.

### DISCLOSURE OF INVENTION

It is the object of the present invention to provide a guide tube for tattoo machine needles, which allows to simply and cost-effectively solve the problem illustrated above, and which is preferably easy to use.

According to the present invention, a guide tube for tattoo machine needles, as defined in claim 1, is provided.

### BRIEF DESCRIPTION OF THE DRAWINGS

The invention will now be described with reference to the appended drawings which illustrate a non-limitative embodiment thereof, in which:
- figure 1 shows a perspective view of a preferred embodiment of the guide tube for tattoo machine needles according to the present invention;
- figures from 2 to 8 show respective variants of the tube in figure 1;
- figure 9 shows a perspective, cross section view of the guide tube in figure 1;
- figure 10 shows a section of a further variant of the tube in figure 1;
- figure 11 is a longitudinal section of the detail in figure 6; and
- figures from 12 to 29 show other variants of the tube in figure 1.

### BEST MODE FOR CARRYING OUT THE INVENTION

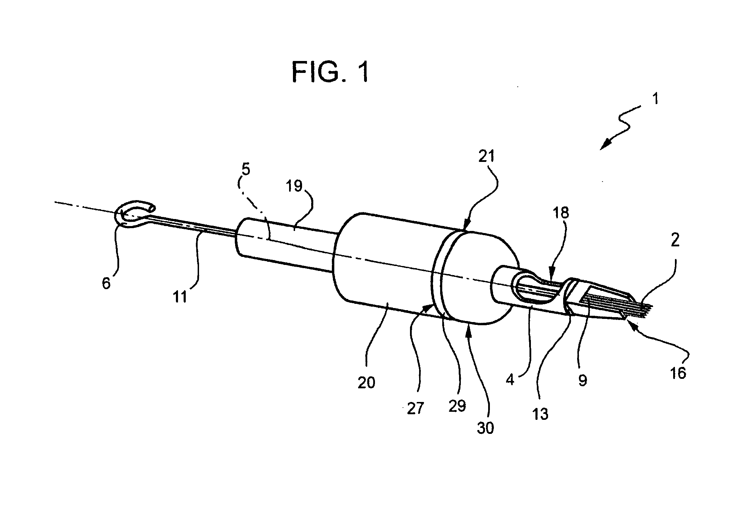

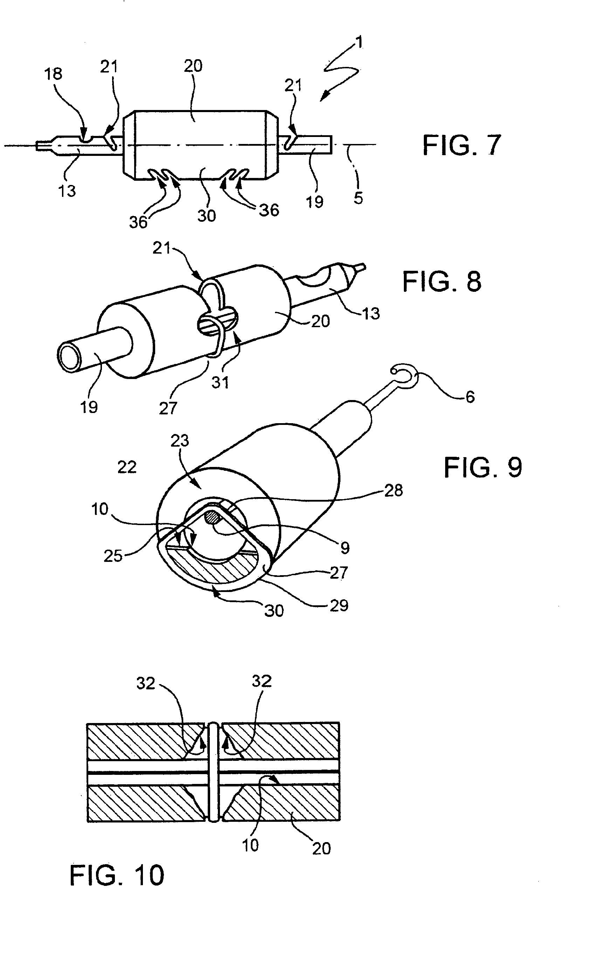

In figures 1 and 9, numeral 1 indicates as a whole a guide tube for the needles 2 of a tattoo machine (not shown).

The needles 2 are fixed, for example, by means of welding, to the end of a front portion 9 of a supporting rod 4, which extends along a rectilinear axis 5. The opposite end is defined by a coupling portion 6, in particular a portion folded as a ring. In the tattoo machine, the portion 6 is coupled in a known manner (not shown) to an actuating device, which translates the rod 4 and thus the needles 2, with reciprocating, rectilinear motion along the axis 5.

The portion 9 engages with clearance a through axial hole 10 of the tube 1. A rear portion 11 of the rod 4 axially protrudes from the tube 1 and ends with portion 6.

The tube 1 comprises a tip 13, which accommodates the needles 2 and the end of portion 9. The tips of the needles 2 protrude from the tube 1 through a frontal outlet 16 of the hole 10. The tip 13 has a side opening 18, which may either communicate with the outlet 16 or not, and which is used to rinse ink from the end of the tip 13.

The tube 1 comprises a tang 19, opposite to the tip 13 and adapted to be fixed to the tattoo machine. The tube 1 then comprises an intermediate portion 20, which is a gripping portion having a diameter larger than those of the tip 13 and of the tang 19. The tip 13, the portion 20 and the tang 19 may be made of either plastic and/or metallic material. Furthermore, they may either be made in one piece or belong to separate pieces fixed to one another. These features related to the construction and the material of the tattoo machine tubes are generally known to a person skilled in the art and therefore will not be described in further detail.

The tube 1 has at least one slot 21, which is transversal to the axis 5, which extends from the top downwards beyond half of the tube 1, i.e. beyond axis 5, so as to intersect the hole 10 so that an elastic band 27 can come into contact with a zone 22 of portion 9 (fig. 9). "Top" is the direction of the side opening 18.

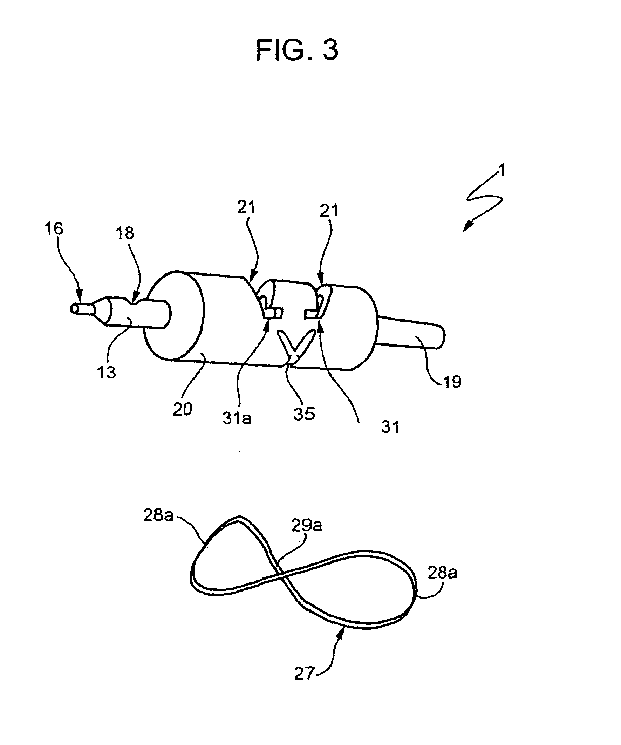

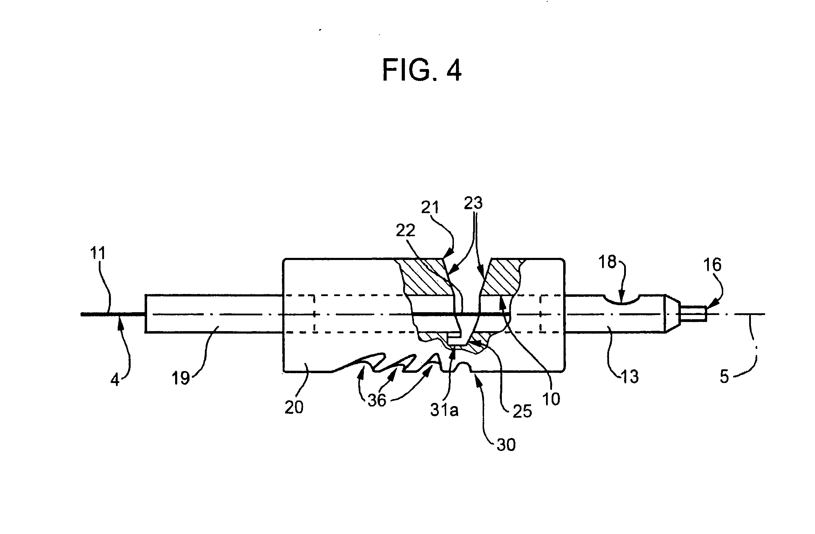

The slot 21 is longitudinally defined by two flanks 23, which face one another and are joined at the bottom of the slot 21 by a joint. In side view, the flanks 23 may be either parallel or converge towards the bottom of the slot 21, so as to have a V-shaped side profile (as shown, for example, in the variants in figures 3 and 4).

Preferably, the elastic band 27 is of annular type and fitted about the tube 1. The elastic band 27 thus comprises: a portion 29 arranged resting on an outer side surface 30 of the tube 1, which is in this case downwards, i.e. diametrically opposite to the inlet of the slot 21; and a portion 28 accommodated in the slot 21 and resting against the tube 1 in two zones 25, on opposite sides of the hole 10 at the bottom of the slot 21.

By virtue of the elastic band 27, the portion 28 presses zone 22 downwards so that the end of the portion 9 and/or the needles 2 slide in contact with the surface of the hole 10 near the outlet 16. In this manner, oscillations or transversal waving of the tips of the needles 2 are limited.

According to the variant shown in figure 8, the longitudinal size of the slot 21 increases near the zones 25, so as to define a cavity 31 which radially withholds the portion 28 and confers the freedom of oscillating in reciprocating motion to the portion 28 together with the rod 4. In this manner, the sliding of the elastic band 27 on the rod 4 is low, and consequently the life of the elastic band 27 increases.

The variant in figure 10 substantially performs the same function: the flanks 23 are sunken so as to form respective recesses 32 about the hole 10.

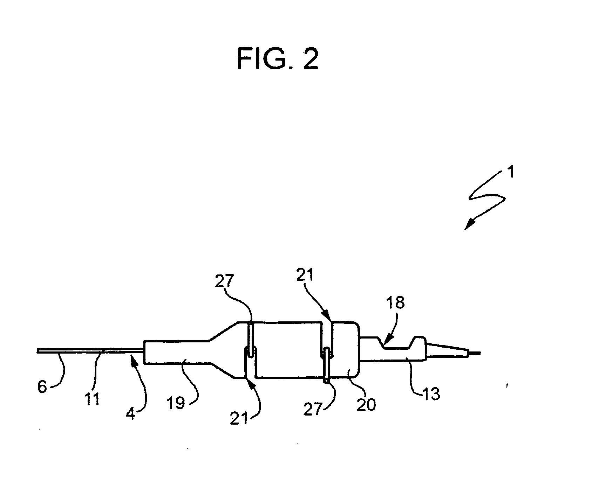

At least two axially distanced slots 21 are provided in the variant in figure 2. Preferably, the inlets of the two slots 21 are made on diametrically opposite parts of axis 5. The front slot 21 exerts an elastic downwards force for pushing the needles 2 down towards the edge of the outlet 16; the rear elastic band (i.e. near the tang 19) exerts an upward force for stabilizing the rod 4 and does not pass much beyond axis 5.

In the variant in figure 3, the inlets of the two slots 21 are made on the same side as the axis 5 (i.e. upwards) and are associated to a single, same elastic band 27. The elastic band 27, in this case, comprises: two portions 28a which respectively engage the slots 21; and a portion 29a which crosses and is arranged in contact with the surface 30, centrally between the slots 21. In order to arrange the portion 29a in a predetermined portion, the surface 30 has a corresponding cross-shaped notch or groove 35 which is engaged by the portion 29a.

The slots 21 widened out longitudinally at the bottom to define respective cavities 31a having L-profile in side view. The cavities 31a radially withhold the portions 28a engaged on the bottom of the slots 21, in addition to leaving a given freedom of movement or deformation to the portions 28a in longitudinal direction.

In the variant of figure 4, the surface 30 has a plurality of positioning and traction notches 36, which are distanced longitudinally from one another and may be selectively engaged by the portion 29 so a to position the portion 29 itself and confer more or less tension to the elastic band 27. Calibrating the tension is appropriate for finding the right compromise between forcing the rod 4 and/or the needles 2 against the surface of the hole 10 (to prevent transversal oscillations of the needles 4) and, at the same time, limiting friction in the tube 1.

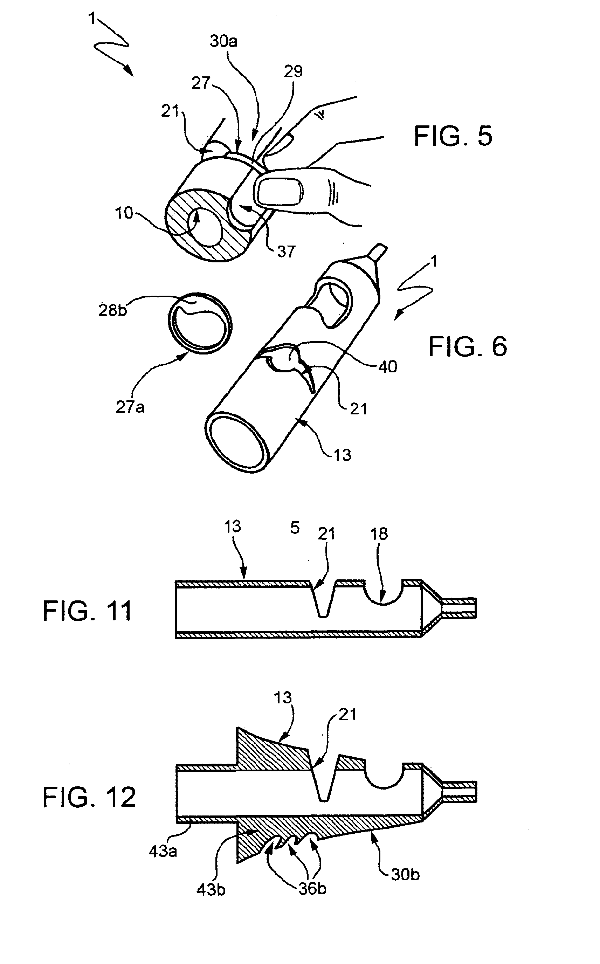

According to the variant in figure 5, the portion 29 extends in contact with an outer surface 30a provided with at least one recess 37: the portion 29 remains distanced from the bottom of the recess 37. In this manner, a finger may be inserted in the recess 37 to grip the portion 29 when either inserting or extracting the elastic 27.

The variant of the figures 6 and 11 tends to make the elastic band more robust in the area which couples with zone 22 of the portion 9 and which could be subjected to friction. Indeed, an elastic band 27a is provided comprising a widened portion 28b with respect to the remaining parts. The portion 28b, in particular, is drop-shaped. Correspondingly, the two edges at the inlet of the slot 21 have a central portion 40 which is concave so as to delimit a widened opening, in order to easily insert the portion 28b of the slot 21. In figure 6 this type of elastic is applied to the tip 13, but could be applied to the portion 20 and/or to the tang 19.

As just mentioned, and as apparent in the attached figures, the slots 21 can be made on any part of the tube 1. When the slot 21 is made on the tip 13 or on the tang 19, the portion 29 of the elastic band 27 may rest on the tip itself 13 (figures from 17 to 20) or, respectively, on the same tang 19 (in manner not shown); or, as shown in the variant in figure 7, the portion 29 may rest on the portion 20, by virtue of at least one positioning notch 36. In the case of figure 7, in order to prevent the elastic band 27 from causing problems to the hands for gripping, the notches or grooves 36 could be deeper (without intersecting the hole 10) so as to sink and conceal portion 29 in the tube 1. Obviously, the slot 21 could be in portion 20, and the notches 36 on the tip 13 and/or on the tang 19.

Figure 12 is a variant of figures 6 and 11: the tip 13 comprises a cylindrical end portion 43a inserted in the portion 20 in manner not shown and an intermediate portion 43b defined by a thicker wall, which axially comes into contact with the portion 20 and joins with the surface 30, so as to improve the gripping of the tube 1 and to consolidate the zone in which the slot 21 is obtained. The intermediate portion 43b also has positioning and traction notches 36b made on a lower surface 30b, i.e. opposite to the inlet of the slot 21. The same principle is applied to the tang 19.

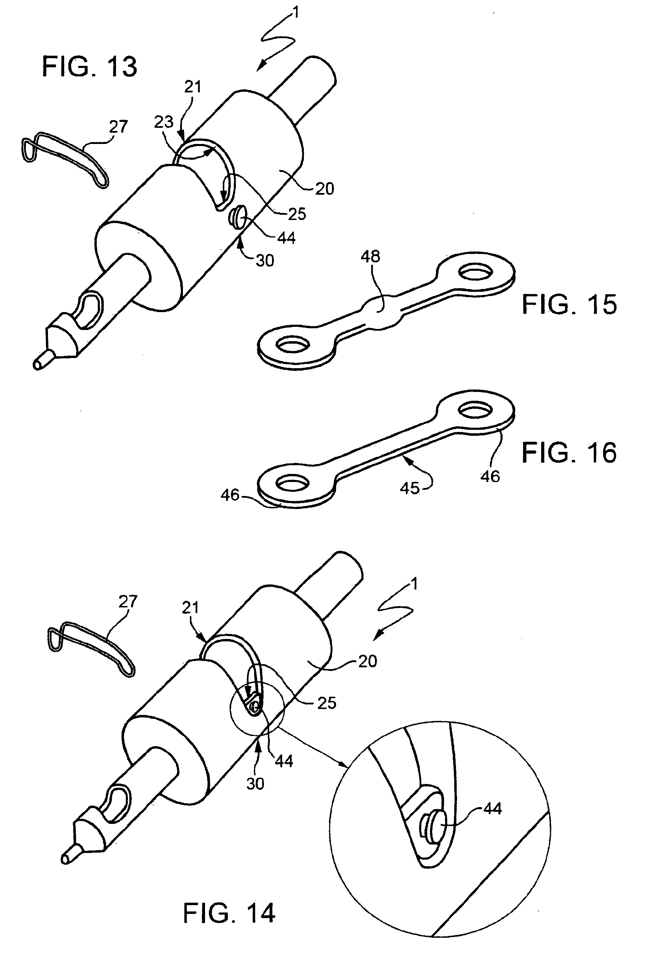

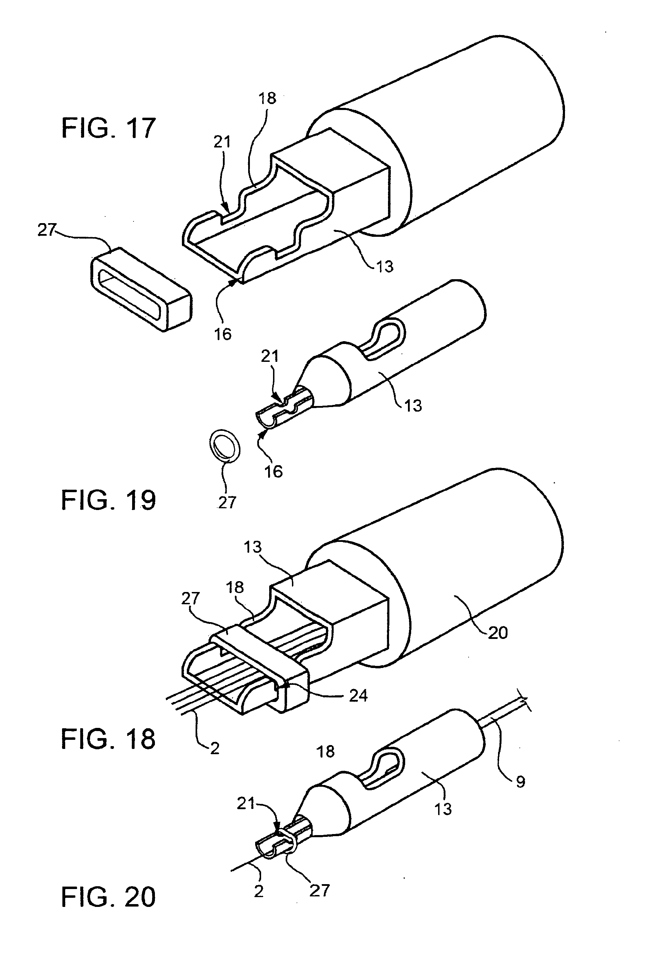

The two variants shown in figures 13 and 14 show how the elastic band 27 may be coupled to two pegs 44 (only one of which is visible), which protrude from the surface 30 or define part of the zones 25 respectively. Alternatively, an open elongated, i.e. not annular, elastic band may be used, with a rectilinear intermediate portion engaging the slot 21 and two ends 46 provided with holes adapted to be engaged by the pegs 44 (figure 16). Possibly, the elastic band 45 may be provided with a widened drop-shaped central portion 48 (figure 15), similar to portion 28b. Several pairs of pegs may be provided arranged along the surface 30 in order to vary the tension.

In the two variants shown in figures from 17 to 20, the slot 21 is made on the end of the tip 13, i.e. near the outlet 16 and/or in the opening 18, to that the elastic band 27 can act directly on the needles and/or on the end of the portion 9.

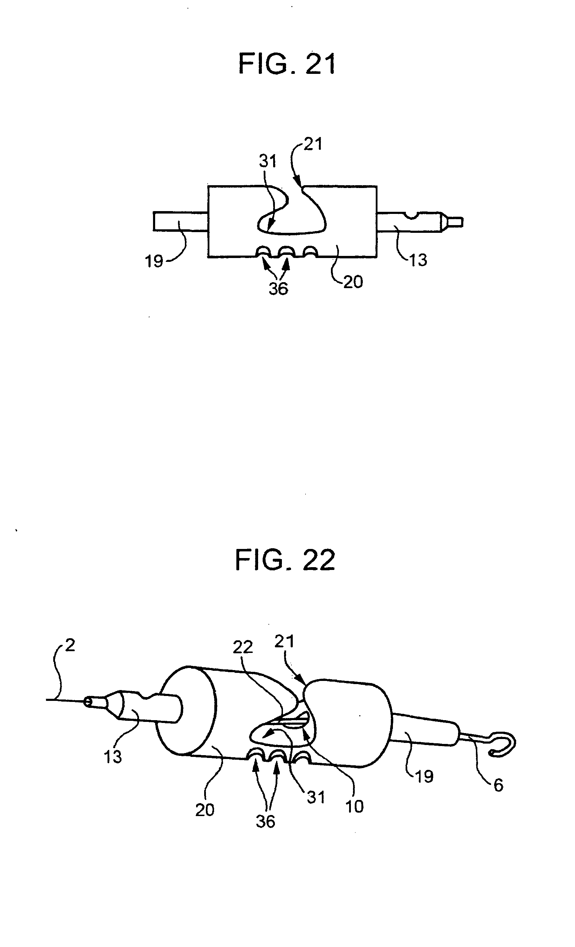

Figures 21 and 22 show a side, perspective view of a variant similar to that shown in figure 8: in particular, the cavity 31 is wider than that shown in figure 8.

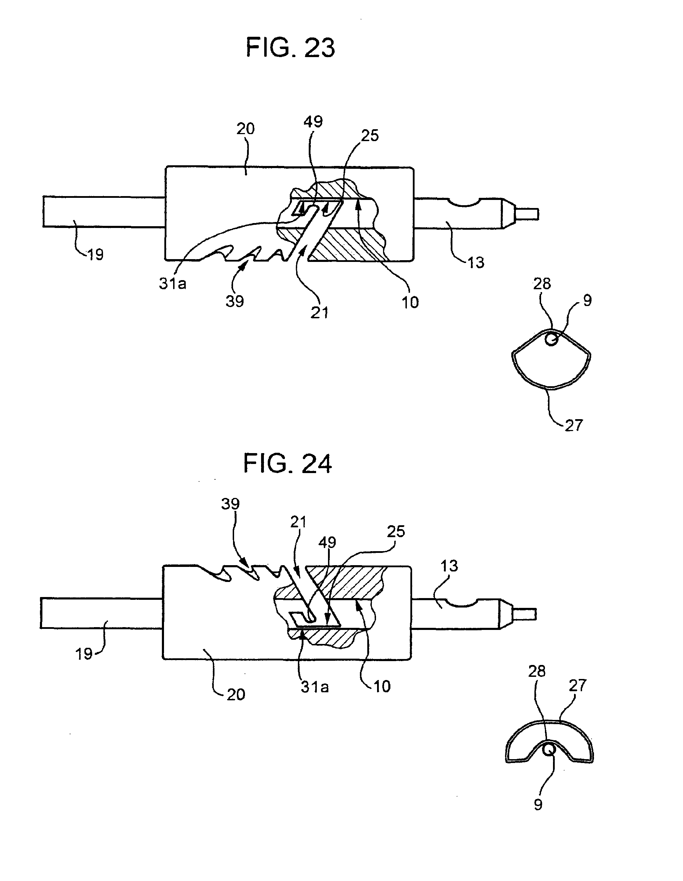

Figures 23 and 24 show two additional shapes with respect to the conformation shown in figure 4: the notches 36 for a portion 29 of the elastic band 27 are made on a surface adjacent to the inlet of the notch 21. Furthermore, on the bottom, the slot 21 defines two teeth 49 which withhold the elastic band 27 in the cavity 31a. In the case of figure 23, the slot 21 is made from the bottom upwards, and the portion 28 of the elastic band 27 is arranged radially between the rod 4 and the bottom of the slot 21, and therefore the elastic band 27 pulls the portion 9 of the rod 4 downwards.

In the case of figure 24, the slot 21 is made from the top downwards and the portion 28 of the elastic band 27 "pushes" the portion 9 of the rod 4 radially downwards.

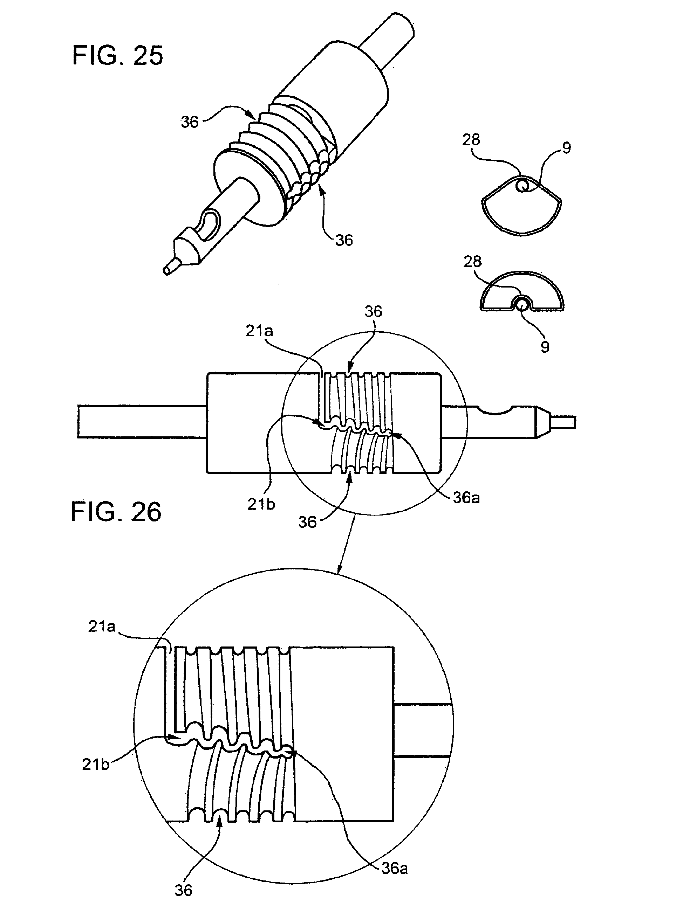

In the variant of figures 25 and 26, the slot 21 has an L-shaped profile with a substantially radial portion 21a and a substantially longitudinal portion 21b. The portion 21b has a series of notches or grooves 36a, preferably made both on the upper face and on the lower face. The notches 36a prevent the portion 28 of the elastic band 27 from going back towards portion 21a. Portion 21b is slightly inclined with respect to axis 5 so as to increase the depth thereof in the hole 10 under the axis 5: in this manner, the tension may be adjusted by moving the portion 28 of the elastic band 27 forwards and backwards between the notches 36a.

The elastic 27 may be inserted in two different configurations, i.e. with the portion 28 which either "pushes" or "pulls" downwards.

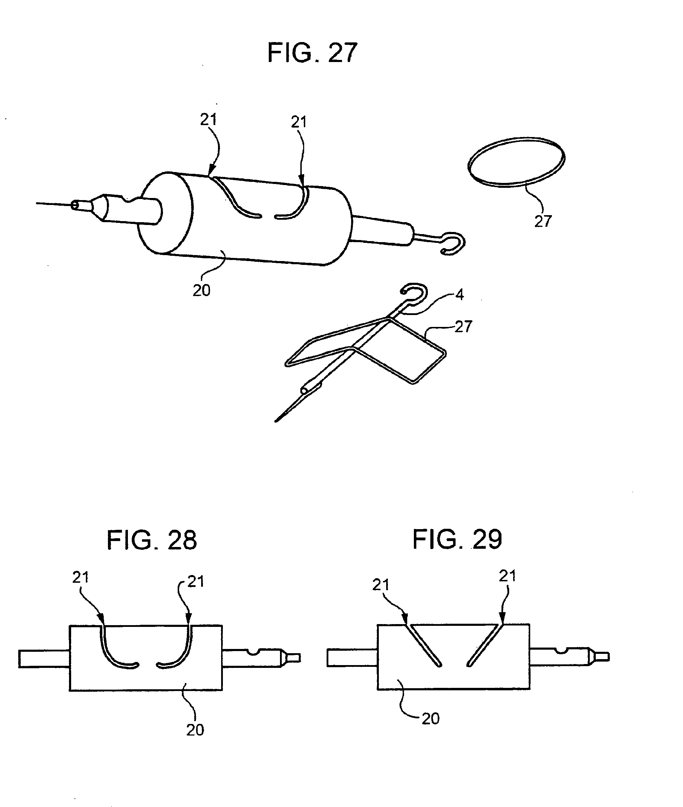

Figures 27 and 28 show a variant of figure 3. The elastic band 27 is inserted in one of the slots 21 without interacting with the surface 30, but only passing through the bottom of the slots 21 themselves. Figure 29 then shows a different shape of the slots 21.

In use, the elastic band 27 cooperates with the surface of the hole 10 to effectively guide the rod 4 in its reciprocating motion. Indeed, the elastic band 27 radially acts on the rod 4 in a zone which is close to the tip of the needles 2, and with a forcing that tends to maintain the needles 2 downwards in contact with the surface of the hole 10 at the outlet 16. Therefore, the elastic band 27 can effectively limit the oscillations or waving of the needles 2 in transversal direction.

Other specific advantages of the invention related for example to ease of use and operation efficacy are apparent from the features described above and shown in the appended drawings.

Finally, from the above, it is apparent that changes and variations can be made to the described tube 1 without departing from the scope of protection of the present invention, as disclosed in the accompanying claims.

The slot 21 may be thin or wide in order to be able to handle the elastic band 27 inside with one's fingers or to be able to insert more elastic bands.

In particular, the size, shape and number of the elastic bands may be different from those shown by way of example; and/or different elasticity may be provided, e.g. soft, medium and hard elastic bands; and/or the reinforcement portion 28b may be other than drop-shaped.

The notches or grooves may be replaced by another positioning system.

The intermediate portion of the tube 1 could have a diameter equal to that of the tang and/or of the tip (e.g. the diameter of the tube could remain substantially constant for the entire or nearly the entire width thereof).

As mentioned for figure 7, the outer surface of the tube 1 may have a groove for entirely accommodating the portion 29 and preventing the elastic band 27 from hindering the gripping of the hand. Furthermore, the zones of the tube 1 in contact with the elastic band 27 should be rounded.

The portion 6 of the rod 4 could have different shape or size in order to be coupled to different types of tattoo machines; similarly, the shape and size of the tang 19 could vary according to the kind of coupling to the tattoo machine.

## Claims

1. A guide tube (1) for tattoo machine needles (2), the guide tube having a through hole (10), which extends along a longitudinal axis (5) and is engaged, in use, by an axially sliding rod (4) which carries said needles (2); **characterized by** having at least one slot (21), which is transversal to said longitudinal axis (5) and intersects said axial through hole (10) to reach beyond said longitudinal axis (5); in use, at least one elastic band (27) being provided in said slot (21) to come in contact against a zone (22) of said rod (4) and force said rod (4) and/or said needles (2) against a surface of said axial through hole (10) close to the outlet (16) of the axial through hole.

2. A tube according to claim 1, **characterized in that** said slot (21) widens out at the bottom so as to define a cavity (31a) for accommodating and/or retaining said elastic band (27).

3. A tube according to claim 2, **characterized in that**, in side view, said cavity has a substantially L-shaped profile.

4. A tube according to claim 1, **characterized in that** said slot (21) is longitudinally delimited by two flanks (23) sunken about the hole (10) so as to form respective recesses (32).

5. A tube according to any one of the preceding claims, **characterized by** comprising positioning means arranged on an external side surface of said guide tube and/or on an inner face of said slot (21).

6. A tube according to claim 5, **characterized in that** said positioning means (36) are configured so as to longitudinally adjust the position of a portion of the elastic band (27).

7. A tube according to claim 6, **characterized in that** said positioning means comprise a series of notches or grooves.

8. A tube according to any one of the preceding claims, **characterized by** having at least two slots (21) longitudinally spaced from each other.

9. A tube according to claim 8, **characterized in that** said slots have respective inlets made on parts diametrically opposite to one another.

10. A tube according to any one of the preceding claims, **characterized in that** the inlet edges of said slot comprise respective concave zones, facing each other and defining a widened central zone (40) therebetween.

## Patentansprüche

1. Führungsrohr (1) für Nadeln (2) einer Tattoomaschine, wobei das Führungsrohr ein Durchgangsloch (10) aufweist, welches sich entlang einer longitudinalen Achse (5) erstreckt und welches, während der Nutzung, durch ein axial verschiebbaren Stab belegt ist, der die Nadeln (2) trägt; **gekennzeichnet dadurch, dass** zumindest ein Schlitz (21) ausgebildet ist, der transversal zu der longitudinalen Achse (5) ist und das axiale Durchgangsloch (10) schneidet, um hinter die longitudinale Achse (5) zu gelangen; wobei während der Benutzung zumindest ein elastisches Band (27) in dem Schlitz (21) bereitgestellt ist, um in Kontakt mit einem Bereich (22) des Stabes (4) zu gelangen und um den Stab (4) und/oder die Nadeln (2) gegen eine Oberfläche des axialen Durchgangsloches (10) in der Nähe des Auslasses (16) des axialen Durchgangsloches zu drücken.

2. Rohr nach Anspruch 1, **dadurch gekennzeichnet, dass** der Schlitz (21) sich an einem Bodenbereich aufweitet, sodass ein Hohlraum (31 a) gebildet wird zur Unterbringung und/oder zur Aufbewahrung des elastischen Bandes (27).

3. Rohr nach Anspruch 2, **dadurch gekennzeichnet, dass**, in einer Seitenansicht, der Hohlraum ein im wesentlichen L-förmiges Profil aufweist.

4. Rohr nach Anspruch 1, **dadurch gekennzeichnet, dass** der Schlitz (21) longitudinal durch zwei Flanken (23) abgegrenzt wird, die zum Loch (10) abgesenkt sind, um so eine entsprechende Ausnehmung (32) zu bilden.

5. Rohr nach einem der vorhergehenden Ansprüche, **dadurch gekennzeichnet, dass** es Positionierungsmittel aufweist, die an einer äußeren Seitenfläche des Führungsrohrs und/oder an einer inneren Fläche des Schlitzes (21) angeordnet sind.

6. Rohr nach Anspruch 5, **dadurch gekennzeichnet, dass** die Positionierungsmittel (36) ausgebildet sind, um longitudinal die Position eines Abschnittes des elastischen Bandes (27) einzustellen.

7. Rohr nach Anspruch 6, **dadurch gekennzeichnet, dass** die Positionierungsmittel einer Reihe von Kerben und Nuten aufweist.

8. Rohr nach einem der vorhergehenden Ansprüche, **dadurch gekennzeichnet, dass** zumindest zwei Schlitze (21) longitudinal voneinander getrennt vorhanden sind.

9. Rohr nach Anspruch 8, **dadurch gekennzeichnet, dass** die Schlitze entsprechende Einlässe aufweisen, die bezüglich des Durchmessers auf gegenüberliegenden Teilen angeordnet sind.

10. Rohr nach einem der vorhergehenden Ansprüche, **dadurch gekennzeichnet, dass** die Einlassränder des Schlitzes entsprechende konkave Bereiche aufweisen, die einander zugewandt sind und dazwischen einen verbreiterten zentralen Bereich (40) aufweisen.

## Revendications

1. Tube de guidage (1) pour des aiguilles de machine à tatouer (2), le tube de guidage ayant un trou débouchant (10), s'étendant le long d'un axe longitudinal (5) et coopère, en service, avec une tige coulissant axialement (4) qui porte lesdites aiguilles (2) ; **caractérisé en ce qu'**il possède au moins une fente (21), qui est transversale audit trou débouchant axial (10) pour dépasser ledit axe longitudinal (5) ; en service, au moins une bande élastique (27) étant mise en place dans ladite fente (21) pour entrer venir au contact d'une zone (22) de ladite tige (4) et forcer ladite tige (4) et/ou lesdites aiguilles (2) contre une surface dudit trou débouchant axial (10) près de la sortie (16) du trou débouchant axial.

2. Tube selon la revendication 1, **caractérisé en ce que** ladite fente (21) s'élargit au fond de façon à définir une cavité (31a) pour recevoir et/ou retenir ladite bande élastique (27).

3. Tube selon la revendication 2, **caractérisé en ce que**, vue de côté, ladite cavité a un profil sensiblement en L.

4. Tube selon la revendication 1, **caractérisé en ce que** ladite fente (21) est délimitée longitudinalement par deux flancs (23) creusés autour du trou (10) de sorte à former des évidements respectifs (32).

5. Tube selon l'une quelconque des revendications précédentes, **caractérisé en ce qu'**il comprend des moyens de positionnement disposés sur une surface du côté extérieur dudit tube de guidage et/ou sur une face interne de ladite fente (21).

6. Tube selon la revendication 5, **caractérisé en ce que** lesdits moyens de positionnement (36) sont configurés de façon à régler longitudinalement la position d'une portion de la bande élastique (27).

7. Tube selon la revendication 6, **caractérisé en ce que** lesdits moyens de positionnement comprennent une série d'encoches ou de rainures.

8. Tube selon l'une quelconque des revendications précédentes, **caractérisé en ce qu'**il possède au moins deux fentes (21) espacées longitudinalement les unes par rapport aux autres.

9. Tube selon la revendication 8, **caractérisé en ce que** lesdites fentes ont des entrées respectives formées sur des parties diamétralement opposées l'une par rapport à l'autre.

10. Tube selon l'une quelconque des revendications précédentes, **caractérisé en ce que** les bords d'entrée de ladite fente comprennent des zones concaves respectives, qui se font face et définissent une zone centrale élargie (40) entre elles.
